# EUROPEAN PATENT APPLICATION

(11) **EP 3 656 473 A1**
(43) Date of publication of application: **27.05.2020**
(21) Application number: 18207379.1
(22) Date of filing: 20.11.2018
(51) Int. Cl.: B01L 3/00, G01N 33/48, G01N 35/08

(54) **MICRODROPLET DETECTION OF CELLS**

(71) Applicant: Lightcast Discovery Limited, Cambridge CB3 0FA (GB)
(72) Inventor: The designation of the inventor has not yet been filed
(74) Representative: Stratagem IPM Limited

(57) **Abstract**

A method of identifying cell types in a biological sample characterised by the steps of:
1) creating from the biological sample aqueous first microdroplets in an immiscible carrier fluid at least some of which are believed to contain cells of the cell type;
2) moving the first microdroplets along a pathway using real or virtual electrowetting electrodes to at least one microdroplet-merging location;
3) moving aqueous second microdroplets containing a reporter system characteristic of the cell type whose presence is being sought along a pathway using real or virtual electrowetting electrodes to the microdroplet merging location;
4) merging the first and second microdroplets at the merging location to produce merged microdroplets and
5) analysing the contents of each merged microdroplet with an optical detection system and detecting an optical signal characteristic of an interaction between the cell and the reporter system.

## Description

This invention relates to a method for the rapid identification, manipulation and selection of cells. It is especially useful for the manipulation of mammalian cells, either from immortalised cell culture samples or directly from tissue samples. It is also especially useful for the rapid and parallel screening of patient samples thought to contain evidence of infections

Devices for manipulating droplets or magnetic beads have been previously described in the art; see for example US6565727, US20130233425 and US20150027889. In the case of droplets, this outcome may be typically achieved by causing the droplets, for example in the presence of an immiscible carrier fluid, to travel through a microfluidic space defined by two opposed walls of a cartridge or microfluidic tubing. Embedded within one or both of these walls are microelectrodes covered with a dielectric layer each of which is connected to an A/C biasing circuit capable of being switched on and off rapidly at intervals to modify the electric field characteristics of the layer. This gives rise to localised directional capillary forces in the vicinity of the microelectrodes which can be used to steer the droplet along one or more predetermined pathways. Such devices, which employ what hereinafter and in connection with the present invention will be referred to as 'real' electrowetting electrodes, are known in the art by the acronym EWOD (Electrowetting on Dielectric) devices.

A variant of this approach, in which the electrowetting forces are optically-mediated, known in the art as optoelectrowetting and hereinafter the corresponding acronym OEWOD, has been disclosed in, for example, US20030224528, US20150298125, US20160158748, US20160160259 and Applied Physics Letters 93 221110 (2008). In particular, the first of these three patent applications discloses various microfluidic devices which include a microfluidic cavity defined by first and second walls and wherein the first wall is of composite design and comprised of substrate, photoconductive and insulating (dielectric) layers. In this single-sided embodiment, between the photoconductive and insulating layers is disposed an array of conductive cells which are electrically isolated from one another and coupled to the photoactive layer and whose functions are to generate corresponding electrowetting electrode locations on the insulating layer. At these locations, the surface tension properties of the droplets can be modified by means of an electrowetting field as described above. The conductive cells may then be temporarily switched on by light impinging on the photoconductive layer. This approach has the advantage that switching is made much easier and quicker although its utility is to some extent still limited by the arrangement of the electrodes. Furthermore, there is a limitation as to the speed at which droplets can be moved and the extent to which the actual droplet pathway can be varied.

Double-sided embodiments of this latter approach has been disclosed in University of California at Berkeley thesis UCB/EECS-2015-119 by Pei. In one example, a cell is described which allows the manipulation of relatively large droplets in the size range 100-500µm using optoelectrowetting across a surface of Teflon AF deposited over a dielectric layer using a light-pattern over electrically-biased amorphous silicon. However, in the devices exemplified the dielectric layer is thin (100nm) and only disposed on the wall bearing the photoactive layer.

Recently in our pending application EP17177204.9 we have described a device and method for manipulating microdroplets which uses optoelectrowetting to provide the motive force. In this OEWOD device, the microdroplets are translocated through a microfluidic space defined by containing walls; for example a pair of parallel plates having the microfluidic space sandwiched therebetween. At least one of the containing walls includes what are hereinafter referred to as 'virtual' electrowetting electrodes locations which are generated by selectively illuminating an area of a semiconductor layer buried within. By selective illumination of the layer with light from a separate light source, a virtual pathway of virtual electrowetting electrode locations can be generated transiently along which the microdroplets can be caused to move. In our corresponding application EP17180391.9, use of this device as an operative part of a nucleic acid sequencer is described.

We have now applied microdroplet methods similar to those underpinning our previously described sequencer to the rapid screening of biological samples containing cells. Thus, according to the present invention there is provided a method of identifying cell types in a biological sample characterised by the steps of:
1) creating from the biological sample aqueous first microdroplets in an immiscible carrier fluid at least some of which are believed to contain cells of the cell type;
2) moving the first microdroplets along a pathway using real or virtual electrowetting electrodes to at least one microdroplet-merging location;
3) moving aqueous second microdroplets containing a reporter system characteristic of the cell type whose presence is being sought along a pathway using real or virtual electrowetting electrodes to the microdroplet merging location;
4) merging the first and second microdroplets at the merging location to produce merged microdroplets and
5) analysing the contents of each merged microdroplet with an optical detection system and detecting an optical signal characteristic of an interaction between the cell and the reporter system.

The method may initially also comprise one or more sorting, culturing and droplet preparation initial steps. These initial steps may comprise one or more of (a) separating cell-containing first microdroplets from a population of microdroplets including both cell-containing and empty microdroplets; (b) culturing the population of microdroplets under conditions which cause cell growth and division before or after initial step (a) is carried out and (c) generating the population of microdroplets by severing microdroplets from the biological sample by application of an electrowetting stretching force. In initial step (c) the microdroplets contain growth medium components are in one embodiment severed into an immiscible carrier fluid comprising an oil thereby creating an emulsion which can be subsequently cultured in initial step (b). In another the microdroplets are severed into air or another gas mixture, for example a mixture of carbon dioxide and nitrogen, and subsequently cultured separately from each other. In some embodiments, it may be advantageous to periodically purge the carrier fluid of gases detrimental to the culturing of the cells.

In one preferred embodiment of initial step (b), culturing of the population of microdroplets is carried out in an emulsion and in the presence of a flowing stream of immiscible carrier fluid such as a hydrocarbon or fluorinated oil, especially a fluorinated oil. This oil may optionally further comprise surfactants and other additives to maintain microdroplet stability. Preferably, the oil also contains low levels of the nutrients and gases required to maintain cell growth during the culturing phase and application of this embodiment causes the nutrient content of the microdroplets to be either periodically or continuously replenished by interfacial diffusion. Alternatively, nutrient and gaseous replenishment can be achieved directly by the merging of secondary aqueous microdroplets containing these components ; for example where the carrier fluid is air or inert gas. In another embodiment the oil is purged of certain dissolved gases in order to provide a hypoxic environment to the cells.

In one preferred embodiment of initial step (b), we have found that in order to maintain cell growth it is necessary to maintain optimum levels of certain atmospheric gases in the microdroplets. Failure to do so can for example cause adverse changes to the pH of the microdroplet medium. Thus in one embodiment initial step (b) the flowing stream of immiscible carrier fluid contains one or more of saturation levels of nitrogen, oxygen or in particular carbon dioxide.

In another embodiment of initial step (b), the contents of the microdroplets are stirred or agitated by application of an electrowetting force at locations where the microdroplets are held. Suitably this is achieved using an optically-mediated electrowetting force delivered for example by an OEWOD structure of the type described below. This approach we believe is also of wider utility and thus in another generally-applicable second aspect of the invention there is provided a method of stirring or otherwise agitating the contents of a microdroplet comprising the steps of (a) locating the microdroplet at a virtual electrowetting electrode location and (b) applying a source of electromagnetic radiation to the location thereby activating the corresponding virtual electrowetting electrode and generating an associated electrowetting force characterised in that the source of electromagnetic radiation is moved around the location to cause a corresponding movement of the microdroplet and a corresponding stirring or agitation of its contents.

Suitably, the source of electromagnetic radiation used in this method corresponds to the second electromagnetic radiation described below and preferably comprises a source of rapidly flashing rotational light describing a circular pathway within or around the periphery of the location. In another preferred embodiment, the movement of the light source may comprise a pathway of one or more lateral motions. In one manifestation, the locations are defined by areas of at least 0.5 microns in diameter and the motion is circular, radial or a mixture of the two. Preferably, the motion is radial to generate corresponding centrifugal mixing of the contents of the microdroplet.

The reporter systems which can be introduced into the first microdroplets in step (4) can in principle be any system which is characteristic of or which may be used to assay the presence of a given cell type or cell behaviour in biological sample. Such reporter systems include, for example, a reporter gene, a cell-surface biomarker or a reporter molecule selective for an enzyme, protein or antibody expressed by cells of the cell type being sought. A related class of reporter system can be a second reporter cell which responds to the presence of relevant material expressed by the cell being sought. Many such assays are known in the prior art suitable candidates for use here will in many cases be apparent to one of ordinary skill. Furthermore it will be appreciated that by introducing a plurality of different reporter systems into the first microdroplets by the merging of one or more second microdroplet types the method may be multiplexed so as to carry out parallel and simultaneous searches for a range different cell types associated with a range of different characteristics and behaviours.

The method of the present invention and it various steps and initial sub-steps can be conveniently carried out using an analytical device of the type below which also generally describes preferred examples of the optical detection system applicable to the method. At its broadest scope this device comprises;
- a sorting component for separating cell-containing microdroplets from empty ones into a population of cell-containing first microdroplets;
- a microdroplet manipulation component for subsequently manipulating the first microdroplets using real or virtual electrowetting electrodes and including:
   ▪ a first zone including a means for introducing a reporter system into each first microdroplet by means of microdroplet merging;
   ▪ an second zone located within or adjacent the first zone in which merged microdroplets are thereafter detected in one or more detection windows and
- an optical detection system for detecting an optical signal from the merged microdroplets arising from an interaction between the reporter system and the cells or an expressed product thereof selected from a brightfield microscope, a darkfield microscope, a means for detecting chemiluminescence, a means for detecting Forster resonance energy transfer or a means for detecting fluorescence.

The sorting component employed in this preferred embodiment is a means for separating microdroplets containing one or more cells (hereinafter 'filled microdroplets) from a larger population some of which are empty. Depending on the type of sorting component chosen, the microdroplets are directed down or towards one of two different microfluidic pathways or receiving locations depending on whether they are filled or empty. In some embodiments, access to one or other of these is controlled by a divider or actuation of an electromechanical gate acting in response to the analysis of each microdroplet in an analytical window. In another preferred embodiment, sorting is accomplished by applying a responding optically-mediated electrowetting force in the analytical window to a stream of the microdroplets so that the chosen ones are pulled into a holding area or array. The rejected microdroplets then remain in the stream and thereafter can be discarded. In another, preferred embodiment, the sorting decision is based on an optical phenomenon; for example brightfield microscopy or by detecting an optical property associated with the cells e.g. the presence of a fluorescent tag or marker. In another embodiment, sorting may be achieved by a dielectrophoretic method in which a temporary electric field is applied in the analytical window to deflect each microdroplet in turn towards one of two different pathways either side of a divider. In one preferred embodiment, the sorting component comprises a first microfluidic channel terminating in analytical chamber; at least two second microfluidic channels connected to the analytical chamber on the downstream side at least one of which carries away the first microdroplets, a light source for illuminating the analytical chamber; a brightfield microscope or fluorescence detector for obtaining data from each illuminated microdroplet in the analytical chamber; at least one OEWOD structure operable to direct the microdroplets down one of the two second channels and a microprocessor adapted to operate the structure(s) in response to a result from an identification algorithm applied to data received from the microscope or fluorescence analyser.

In one embodiment, the device further comprises a culturing-component which is either an integral component of the device itself or separately located before or after the sorting component; preferably after the sorting component. Here, the microdroplets are held whilst any cells contained within are cultured so as to stimulate cell division and growth. Suitably, the culturing component comprises a vessel in which the microdroplets are held under optimal culturing conditions; typically from 6 to 72 hours at a temperature in the range above 25°C (e.g. 25 to 40°C) and inlet port for introducing the microdroplets thereinto. In one embodiment, the culturing component further comprises a thermostatically-controlled heater and optionally a timer which controls filling and discharge cycles for the vessel. In one embodiment, the contents of the vessel comprises an emulsion of microdroplets in an immiscible carrier fluid and the vessel further comprises an inlet and outlet through which carrier fluid can be passed allowing it to be replaced over time. In one embodiment, the immiscible carrier fluid is preferably a fluorocarbon oil such as HFE7500, HFE7700 or FC-40. Such oils suitably further contain surfactants and other additives to maintain microdroplet stability and low levels of the nutrients and gases required to maintain growth.

In one embodiment, of particular utility where the weight ratio of aqueous microdroplets to oil is low, there is a tendency for the microdroplets to shrink over time; a phenomenon which can lead to a loss of reactivity within them. One preferred way of counteracting this effect is to use an oil which has been hydrated. Since generally the oils described above do not have a high capacity for dissolving water, hydration is suitably achieved by creating micelles or secondary microdroplets of water or aqueous buffer within the oil phase. This buffer may have a composition which is the same as or different to that of the microdroplets themselves. In one embodiments these micelles or secondary microdroplet may contain up to five times the salt content of the microdroplets themselves and optionally contain glycerol. Typically these micelles and secondary microdroplets are an order of magnitude smaller.

In one embodiment, the vessel further comprises a surface provided with a plurality of locations at which the microdroplets can be located and agitated to stir their contents using optically mediated electrowetting forces; also as described above.

The preferred device further comprises a sample preparation component either integral with the device itself or separately located upstream of the culturing component which comprises a means for creating an emulsion of the microdroplets in an immiscible carrier fluid from an aliquot of the biological sample.

Preferably, this sample preparation component includes a severing means for severing microdroplets from the biological sample into the carrier fluid which comprises at least one location where an electrowetting stretching force is applied to the biological sample. In one preferred embodiment the severing means comprises:
- a first electrowetting location adapted to receive the biological sample;
- at least one second electrowetting location arranged so that the first and second electrowetting electrode locations define a pathway along which microdroplets severed from the sample can be transported using directional electrowetting forces;
- an AC drive circuit arranged at the first electrowetting location and comprised of either an electrode and an associated AC electrical circuit or a semiconductor zone activated by the impingement of electromagnetic light thereon and
- a DC charging circuit arranged at the first electrowetting location and adapted to electrostatically charge the surface of the biological sample.

In one embodiment, the severing means further comprises a control circuit for switching between the drive and charging circuits which are suitably AC and DC circuits respectively. In another embodiment, the severing means further comprises an analyser for analysing the contents of each microdroplet produced from the biological sample. In this respect, the biological sample can be in the form of any aqueous material such as blood, plasma, sputum, urine or material derived from tissue biopsies. Further information about suitable severing means can be found in our co-pending application EP18201162.7 to which the reader is directed. It will be readily appreciated that microdroplet-severing method associated with this severing means can form the basis of carrying out initial step (c) above.

Turning to the microdroplet manipulation component which is used to subsequently manipulate the first microdroplets produced by the sorting component, this is suitably a microfluidic chip comprised of a first zone, a second zone and an optical detection system comprised of real or virtual electrowetting electrodes and linked together by one or more microfluidic pathways along which the first microdroplets are driven by pneumatic and/or electrowetting forces. Suitably, the electrowetting electrodes are virtual and established at locations in one or more OEWOD structures. Generally, this is the preferred way of manipulating the microdroplets in the method and in one embodiment these OEWOD structures are comprised of:
- a first composite wall comprised of:
   ▪ a first transparent substrate
   ▪ a first transparent conductor layer on the substrate having a thickness in the range 70 to 250nm;
   ▪ a photoactive layer activated by electromagnetic radiation in the wavelength range 400-1000nm on the conductor layer having a thickness in the range 300-1000nm and
   ▪ a first dielectric layer on the conductor layer having a thickness in the range 120 to 160nm;
- a second composite wall comprised of:
   ▪ a second substrate;
   ▪ a second conductor layer on the substrate having a thickness in the range 70 to 250nm and
   ▪ optionally a second dielectric layer on the conductor layer having a thickness in the range 120 to 160nm
      wherein the exposed surfaces of the first and second dielectric layers are disposed at least 10µm apart to define a microfluidic space adapted to contain microdroplets; and either of the dielectric layers are coated with a biocompatible coating or an anti-fouling coating. Optionally there may be an interstitial layer of silica of thickness 1-10nm in between the dielectric layers and the anti-fouling/biocompatible coatings;
- an A/C source to provide a voltage across the first and second composite walls connecting the first and second conductor layers;
- at least one source of second electromagnetic radiation having an energy higher than the bandgap of the photoexcitable layer adapted to impinge on the photoactive layer to induce corresponding virtual electrowetting electrode locations on the surface of the first dielectric layer and
- means for manipulating the points of impingement of the electromagnetic radiation on the photoactive layer so as to vary the disposition of the virtual electrowetting electrode locations thereby creating at least one optically-mediated electrowetting pathway along which the microdroplets may be caused to move.

In one embodiment, the first and second walls of these structures are transparent with the microfluidic space sandwiched in-between. In another, the first substrate and first conductor layer are transparent enabling light from the source of electromagnetic radiation (for example multiple laser beams, a lamp or a LED) to impinge on the photoactive layer. In another, the second substrate, second conductor layer and second dielectric layer are transparent so that the same objective can be obtained. In yet another embodiment, all these layers are transparent.

Suitably, the first and second substrates are made of a material which is mechanically strong for example glass metal or an engineering plastic. In one embodiment, the substrates may have a degree of flexibility. In yet another embodiment, the first and second substrates have a thickness in the range 100-1000µm.

The first and second conductor layers are located on one surface of the first and second substrates and are typically have a thickness in the range 70 to 250nm, preferably 70 to 150nm. In one embodiment, at least one of these layers is made of a transparent conductive material such as Indium Tin Oxide (ITO), a very thin film of conductive metal such as silver or a conducting polymer such as PEDOT or the like. these layers may be formed as a continuous sheet or a series of discrete structures such as wires. Alternatively, the conductor layer may be a mesh of conductive material with the electromagnetic radiation being directed between the interstices of the mesh.

The photoactive layer is suitably comprised of a semiconductor material which can generate localised areas of charge in response to stimulation by the source of the second electromagnetic radiation. Examples include hydrogenated amorphous silicon layers having a thickness in the range 300 to 1000nm. In one embodiment, the photoactive layer is activated by the use of visible light.

The photoactive layer in the case of the first wall and optionally the conducting layer in the case of the second wall are coated with a dielectric layer which is typically in the thickness range from 120 to 160nm. The dielectric properties of this layer preferably include a high dielectric strength of >10^7 V/m and a dielectric constant of >3. Preferably, it is as thin as possible consistent with avoiding dielectric breakdown. In one embodiment, the dielectric layer is selected from alumina, silica, hafnia or a thin non-conducting polymer film.

In another embodiment of these structures, at least the first dielectric layer, preferably both, are coated with an anti-fouling layer to assist in the establishing the desired microdroplet/carrier fluid/surface contact angle at the various virtual electrowetting electrode locations, and additionally to prevent the contents of the microdroplets adhering to the surface and being diminished as the microdroplet is moved through the chip. If the second wall does not comprise a second dielectric layer, then the second anti-fouling layer may be applied directly onto the second conductor layer. For optimum performance, the anti-fouling layer should assist in establishing a microdroplet/carrier fluid/surface contact angle that should be in the range 50-170° when measured as an air-liquid-surface three-point interface at 25°C. In one embodiment, these layer(s) have a thickness of less than 50nm and are typically a monomolecular layer. In another, these layers are comprised of a polymer of an acrylate ester such as methyl methacrylate or a derivative thereof substituted with hydrophilic groups; e.g. alkoxysilyl. Preferably, either or both of the anti-fouling layers are hydrophobic to ensure optimum performance. In some embodiments an interstitial layer of silica may be interposed between the anti-fouling coating and the dielectric layer in order to provide a chemically compatible bridge.

The first and second dielectric layers, and therefore the first and second walls, define a microfluidic space which is at least 10µm in width and in which the microdroplets are contained. Preferably, before they are contained, the microdroplets themselves have an intrinsic diameter which is more than 10% greater, suitably more than 20% greater, than the width of the microdroplet space. By this means, on entering the chip the microdroplets are caused to undergo compression leading to enhanced electrowetting performance through e.g. a better microdroplet merging capability.

In one embodiment the first and second dielectric layers are coated with a hydrophobic coating such a fluorosilane.

In another embodiment, the microfluidic space includes one or more spacers for holding the first and second walls apart by a predetermined amount. Options for spacers includes beads or pillars, ridges created from an intermediate resist layer which has been produced by photopatterning. Alternatively, deposited material such as silicon oxide or silicon nitride may be used to create the spacers. Various spacer geometries can be used to form narrow channels, tapered channels or partially enclosed channels which are defined by lines of pillars. By careful design, it is possible to use these spacers to aid in the deformation of the microdroplets, subsequently perform microdroplet splitting and effect operations on the deformed microdroplets. Similarly these spacers can be used to physically separate zones of the chip to prevent cross-contamination between droplet populations, and to promote the flow of droplets in the correct direction when loading the chip under hydraulic pressure.

The first and second walls are biased using a source of A/C power attached to the conductor layers to provide a voltage potential difference therebetween; suitably in the range 10 to 50 volts.

These preferred OEWOD structures are typically employed in association with a source of second electromagnetic radiation having a wavelength in the range 400-1000nm and an energy higher than the bandgap of the photoexcitable layer. Suitably, the photoactive layer will be activated at the virtual electrowetting electrode locations where the incident intensity of the radiation employed is in the range 0.01 to 0.2 Wcm⁻². The source of electromagnetic radiation is, in one embodiment, pixelated so as to produce corresponding photoexcited regions on the photoactive layer which are also pixelated. By this means, pixelated virtual electrowetting electrode locations are induced on the first dielectric layer.

Where the source of electromagnetic radiation is pixelated it is suitably supplied either directly or indirectly using a reflective screen such as a digital micromirror device (DMD) illuminated by light from LEDs or other lamps. This enables highly complex patterns of virtual electrowetting electrode locations to be rapidly created and destroyed on the first dielectric layer thereby enabling the microdroplets to be precisely steered along essentially any virtual pathway using closely-controlled electrowetting forces. This is also especially advantageous where there is a requirement for the chip to manipulate many thousands of such microdroplets simultaneously along multiple pathways. Such electrowetting pathways can be viewed as being constructed from a continuum of virtual electrowetting electrode locations on the first dielectric layer.

The points of impingement of the sources of electromagnetic radiation on the photoactive layer can be any convenient shape including the conventional circular or annular. In one embodiment, the morphologies of these points are determined by the morphologies of the corresponding pixelations and in another correspond wholly or partially to the morphologies of the microdroplets once they have entered the microfluidic space. In one preferred embodiment, the points of impingement and hence the electrowetting electrode locations may be crescent-shaped and orientated in the intended direction of travel of the microdroplet. Suitably the electrowetting electrode locations themselves are smaller than the microdroplet surface adhering to the first wall and give a maximal field intensity gradient across the contact line formed between the droplet and the surface dielectric.

In one embodiment of the OEWOD structure, the second wall also includes a photoactive layer which enables virtual electrowetting electrode locations to also be induced on the second dielectric layer by means of the same or different source of electromagnetic radiation. The addition of a second dielectric layer enables transition of the wetting edge of a microdroplet from the upper to the lower surface of the structure, and the application of more electrowetting force to each microdroplet.

The first zone which forms part of the preferred device is in one embodiment, a holding reservoir comprising an inlet for introducing the first microdroplets and an outlet attached by an electrowetting pathway to the second zone. The first zone further includes a port for introducing a reporter system which in one suitable embodiment is a second inlet for introducing second aqueous microdroplets containing a reporter system designed to identify the nature of the cells contained in the first microdroplets. In one embodiment the reservoir further includes an array of locations where the first microdroplets can be held whilst the second microdroplets are driven over them; in the process causing a degree of merging of the first and second microdroplets. The merged first/second microdroplets (hereinafter' merged microdroplets') can then be held at the locations until the reporter system has interacted sufficiently with cells to subsequently generate an optimum optical signal at which time they are transported to the second zone by electrowetting. In some instances it may be desirable to monitor the growth of the optical signal using time-resolved measurements. In one preferred embodiment, a single zone encompassing the duties of both the first and second zones is employed; for example by detecting the merged microdroplets at the merging locations referred to above.

The second zone is suitably comprised of one or more detection windows through which the merged microdroplets can be analysed using an optical detection system. In one embodiment. the second zone is a transparent section of the chip. In another embodiment, the optical detection system is one designed to detect an optical system from the microdroplets arising from an interaction between the reporter system and the cells or an expressed product thereof. Suitably, the optical detection system is selected from a brightfield microscope, darkfield microscope, a means for detecting chemiluminescence, a means for detecting Forster resonance energy transfer or a means for detecting fluorescence. In one embodiment, the detection system also includes a source of light to illuminate the merged microdroplets and/or a microprocessor for receiving a signal from one of the detectors and providing data to a user in the form of, for example, a visual display or count. In one embodiment, the microprocessor is further adapted by means of a feedback loop to control one or more of the performance of the sorting component; the rate of introduction of the first microdroplets into the first zone and the rate of merging of the first and reporter system-containing microdroplets in response to a signal detected by the optical detection system.

## Claims

1. A method of identifying cell types in a biological sample **characterised by** the steps of:
1) creating from the biological sample aqueous first microdroplets in an immiscible carrier fluid at least some of which are believed to contain cells of the cell type;
2) moving the first microdroplets along a pathway using real or virtual electrowetting electrodes to at least one microdroplet-merging location;
3) moving aqueous second microdroplets containing a reporter system characteristic of the cell type whose presence is being sought along a pathway using real or virtual electrowetting electrodes to the microdroplet merging location;
4) merging the first and second microdroplets at the merging location to produce merged microdroplets and
5) analysing the contents of each merged microdroplet with an optical detection system and detecting an optical signal characteristic of an interaction between the cell and the reporter system.

2. A method as claimed in claim 1 **characterised in that** it includes the initial step (a) of separating cell-containing first microdroplets from a population of microdroplets including both cell-containing and empty microdroplets.

3. A method as claimed in claim1 or claim 2 **characterised in that** it includes the initial step (b) of culturing the population of microdroplets under conditions which cause cell growth and division.

4. A method claimed in claim 1 **characterised in that** the cell-containing first microdroplets are further sub-sorted by the output of measuring the state of the cells with an optical detection system

5. A method as claimed in any one of the preceding claims **characterised in that** it includes the initial step (c) of generating the population of microdroplets by severing microdroplets from the biological sample by application of an electrowetting stretching force.

6. A method as claimed in claim 4 **characterised in that** the microdroplets are severed into the immiscible carrier fluid comprising a hydrocarbon or silicone oil.

7. A method as claimed in any one of the preceding claims **characterised in that** the immiscible carrier fluid is a fluorocarbon oil which has been optionally hydrated with aqueous micelles or secondary microdroplets.

8. A method as claimed in claim 3 **characterised in that** initial step (b) is carried out by contacting an emulsion of the population of microdroplets in the carrier fluid with a flow of carrier fluid containing cell-culturing nutrients and/or dissolved gas.

9. A method as claimed in claim 7 **characterised in that** the dissolved gases consist of one or more of oxygen, nitrogen and carbon dioxide.

10. A method as claimed in claim 7 **characterised in that** the oil is periodically purged of gases detrimental to the culturing of the cells.

11. A method as claimed in any one of claims 3 to 8 **characterised in that** the contents of the microdroplets in initial step (b) are stirred or agitated by application of an optically-mediated electrowetting force at virtual electrowetting electrode locations where the microdroplets are held.

12. A method as claimed in any one of the preceding claims **characterised in that** the reporter system is a reporter gene, cell-surface biomarker or a reporter molecule selective for an enzyme or antibody expressed by cells of the cell type being sought.

13. A method as claimed in any one of the preceding claims that the reported system is a luminescent reporter cell which reacts selectively to the presence of an enzyme or antibody expressed by cells of the cell type being sought

14. A method as claimed in any one of the preceding claims **characterised in that** the optical detection system is one of a brightfield microscope, a darkfield microscope, a means for detecting chemiluminescence, a means for detecting Forster resonance energy transfer or a means for detecting fluorescence.

15. A method as claimed in any one of the preceding claims characterised that microdroplets are transported between at least two of steps (1) to (5) or initial steps thereof using at least OEWOD structure adapted to generate a pathway of virtual electrowetting electrodes using electromagnetic radiation and optionally provided with an anti-fouling and/or biocompatible coating.
